# EUROPEAN PATENT APPLICATION

(11) **EP 4 552 667 A1**
(43) Date of publication of application: **14.05.2025**
(21) Application number: 23867799.1
(22) Date of filing: 20.04.2023
(51) Int. Cl.: A61M 5/00, A61M 5/31

(54) **PREFILLED SYRINGE, DISTAL END MEMBER, SYRINGE OUTER CYLINDER, AND MANUFACTURING METHOD FOR PREFILLED SYRINGE**

(30) Priority: 20.09.2022 JP 2022148838
(71) Applicant: Terumo Kabushiki Kaisha, Tokyo 151-0072 (JP)
(72) Inventor: OKIHARA, Hitoshi, Fujinomiya-shi, Shizuoka 418-0004 (JP)
(74) Representative: Prüfer & Partner mbB Patentanwälte · Rechtsanwälte
(86) International application number: PCT/JP2023/015746
(87) International publication number: WO 2024/062668

(57) **Abstract**

A prefilled syringe (10) includes a syringe outer cylinder (16), a first gasket (18), a freeze-dried agent (12), a distal member (22), and a cap (24).

A displacement of the distal member (22) from a first state in which the distal member (22) is temporarily mounted on a distal portion of the syringe outer cylinder (16) to a second state in which the first chamber (52) is sealed by the distal member (22) is completed.

The first state is a state in which the first convex portion (64) is fitted into the first concave portion (92) and the first chamber (52) communicates with an outside via a gap between the syringe outer cylinder (16) and the cylindrical portion (82) or a gap provided in the distal member (22). The second state is a state in which the first convex portion (64) is fitted into the second concave portion (94) and the second convex portion (66) is fitted into the first concave portion (92).

## Description

### Technical Field

The present invention relates to a prefilled syringe, a distal member, a syringe outer cylinder, and a method for manufacturing a prefilled syringe.

### Background Art

For example, Japanese Patent No. 6182073 discloses a prefilled syringe in which a powder agent is accommodated in an accommodation chamber of a syringe outer cylinder. The prefilled syringe includes a gasket slidably disposed in the syringe outer cylinder and a distal member attached to a distal portion of the syringe outer cylinder such that the powder agent is sealed in the accommodation chamber of the syringe outer cylinder.

### Summary of Invention

Incidentally, the above-described prefilled syringe is manufactured, for example, by powdering a freeze-dried agent manufactured in advance in a vial and transferring the powdered freeze-dried agent from the vial to the accommodation chamber of the syringe outer cylinder. However, in this case, it is necessary to transfer the powder agent from the vial to the syringe outer cylinder.

If the freeze-dried agent can be directly manufactured in the accommodation chamber of the syringe outer cylinder, a powder agent transfer step is not required, which is efficient. To manufacture the freeze-dried agent in the accommodation chamber of the syringe outer cylinder, it is necessary to freeze a liquid agent in a state where the accommodation chamber is filled with the liquid agent, dry (freeze-dry) a freezing agent in a decompression chamber to obtain the freeze-dried agent, and then attach the distal member to the syringe outer cylinder to seal the accommodation chamber. However, in this case, it is necessary to provide a large attachment device in the decompression chamber for attaching the distal member to the syringe outer cylinder, which is not realistic. Note that, if the distal member is mounted on the syringe outer cylinder and the accommodation chamber is sealed in advance before the syringe outer cylinder is placed in the decompression chamber, the freezing agent cannot be sublimated during freeze-drying.

An object of the present invention is to solve the above-described problems.

(1) A first aspect of the present invention is a prefilled syringe including a syringe outer cylinder, a gasket slidably disposed in the syringe outer cylinder, a freeze-dried agent accommodated in an accommodation chamber of the syringe outer cylinder, a distal member mounted on a distal portion of the syringe outer cylinder, and a cap configured to be attached to and detached from the distal member, in which the distal member includes a cylindrical portion into which the distal portion of the syringe outer cylinder is inserted, and a nozzle located inside the cylindrical portion and having a lumen communicating with the accommodation chamber, the cap seals a distal opening of the nozzle in a state of being mounted on the distal member, a first convex portion and a second convex portion located in a proximal direction of the syringe outer cylinder relative to the first convex portion are provided on a distal portion of an outer peripheral surface of the syringe outer cylinder, a first concave portion and a second concave portion located in a distal direction of the syringe outer cylinder relative to the first concave portion are provided in an inner peripheral surface of the cylindrical portion, a displacement of the distal member from a first state in which the distal member is temporarily mounted on the distal portion of the syringe outer cylinder to a second state in which the accommodation chamber is sealed by the distal member is completed, the first state is a state in which the first convex portion is fitted into the first concave portion and the accommodation chamber is in communication with an outside via a gap between the syringe outer cylinder and the cylindrical portion or a gap provided in the distal member, and the second state is a state in which the first convex portion is fitted into the second concave portion and the second convex portion is fitted into the first concave portion.
(2) The prefilled syringe according to item (1), in which the second concave portion is a through-hole, and the gap provided in the distal member is the through-hole.
(3) The prefilled syringe according to the item (1) or (2), in which a first engagement portion is provided on the distal portion of the outer peripheral surface of the syringe outer cylinder, a second engagement portion configured to engage with the first engagement portion when the distal member is mounted on the syringe outer cylinder is provided in the inner peripheral surface of the cylindrical portion, one of the first engagement portion and the second engagement portion includes a protruding portion, and the other of the first engagement portion and the second engagement portion includes a groove portion that is configured to allow the protruding portion to be inserted therein and that extends along an axial direction of the syringe outer cylinder.
(4) The prefilled syringe according to item (3), in which an inner surface of the groove portion has a stopper surface that faces the axial direction of the syringe outer cylinder, and the protruding portion is abutted to or close to the stopper surface.
(5) The prefilled syringe according to any one of items (1) to (4), in which the distal member includes a tubular connection section that covers a distal portion of the nozzle from radially outside, the connection section includes a first screw portion, and the cap includes a second screw portion to be screwed to the first screw portion, and a sealing member configured to seal the distal opening of the nozzle in a state in which the second screw portion is fastened to the first screw portion.
(6) The prefilled syringe according to any one of items (1) to (5), in which a proximal portion of the nozzle is located in a lumen of the cylindrical portion, the distal member includes an annular packing disposed in a liquid-tight manner on an outer peripheral surface of the nozzle, and the packing is inserted into a lumen of the syringe outer cylinder to seal a distal opening of the syringe outer cylinder.
(7) The prefilled syringe according to item (6), in which a proximal surface of the packing has a slope inclined in a distal direction of the nozzle toward an axis of the nozzle, and a distal surface of the gasket has a distal slope inclined radially inward toward the distal direction of the syringe outer cylinder.
(8) The prefilled syringe according to item (7), in which the distal slope of the gasket is configured to come into contact with the entire slope of the packing.
(9) The prefilled syringe according to any one of items (1) to (8), in which the gasket is a first gasket, the prefilled syringe includes a second gasket that is disposed in a proximal direction relative to the first gasket in the lumen of the syringe outer cylinder and that is configured to slide in the axial direction of the syringe outer cylinder, and a plunger configured to press the second gasket in a distal direction, in an initial state of the prefilled syringe, a liquid chamber filled with a liquid agent is formed between the first gasket and the second gasket, the syringe outer cylinder has a bypass structure configured to guide the liquid agent to the accommodation chamber, and the liquid agent is prevented from flowing into the bypass structure by the first gasket in the initial state of the prefilled syringe, and is guided to the accommodation chamber by movement of the first gasket in the distal direction of the syringe outer cylinder and the accommodation chamber and the liquid chamber communicating with each other via the bypass structure.
(10) The prefilled syringe according to item (9), in which the syringe outer cylinder includes a tubular body, and a flange portion protruding radially outward from a proximal portion of the tubular body and extending in a circumferential direction, the prefilled syringe includes a finger grip provided on a proximal portion of the syringe outer cylinder and configured to allow a user to hook a finger thereon, a male screw portion is formed on an outer peripheral surface of the plunger, the flange portion includes a first restriction portion, the finger grip includes a second restriction portion configured to restrict rotation of the syringe outer cylinder in a circumferential direction with respect to the syringe outer cylinder by abutment of the first restriction portion to the second restriction portion, and a plunger engagement portion having a projection to be screwed to the male screw portion, and the plunger moves in the distal direction with respect to the syringe outer cylinder by rotating in a circumferential direction of the finger grip.
(11) A second aspect of the present invention is a distal member of a prefilled syringe, in which the prefilled syringe includes a syringe outer cylinder, a gasket slidably disposed in the syringe outer cylinder, a freeze-dried agent accommodated in an accommodation chamber of the syringe outer cylinder, and a cap configured to be attached to and detached from the distal member, a first convex portion and a second convex portion located in a proximal direction of the syringe outer cylinder relative to the first convex portion are provided on a distal portion of an outer peripheral surface of the syringe outer cylinder, the distal member includes a cylindrical portion into which the distal portion of the syringe outer cylinder is inserted, and a nozzle located inside the cylindrical portion and having a lumen communicating with the accommodation chamber, in which the cap seals a distal opening of the nozzle in a state of being mounted on the distal member, a first concave portion and a second concave portion located in a distal direction of the syringe outer cylinder relative to the first concave portion are provided in an inner peripheral surface of the cylindrical portion, a displacement of the distal member from a first state in which the distal member is temporarily mounted on the distal portion of the syringe outer cylinder to a second state in which the accommodation chamber is sealed by the distal member is completed, the first state is a state in which the first convex portion is fitted into the first concave portion and the accommodation chamber is in communication with an outside via a gap between the syringe outer cylinder and the cylindrical portion or a gap provided in the distal member, and the second state is a state in which the first convex portion is fitted into the second concave portion and the second convex portion is fitted into the first concave portion.
(12) A third aspect of the present invention is a syringe outer cylinder of a prefilled syringe, in which the prefilled syringe includes a distal member mounted on a distal portion of the syringe outer cylinder, a gasket slidably disposed in the syringe outer cylinder, a freeze-dried agent accommodated in an accommodation chamber of the syringe outer cylinder, and a cap configured to be attached to and detached from the distal member, the distal member includes a cylindrical portion into which the distal portion of the syringe outer cylinder is inserted, and a nozzle located inside the cylindrical portion and having a lumen communicating with the accommodation chamber, the cap seals a distal opening of the nozzle in a state of being mounted on the distal member, a first concave portion and a second concave portion located in a distal direction of the syringe outer cylinder relative to the first concave portion are provided in an inner peripheral surface of the cylindrical portion, a first convex portion and a second convex portion located in a proximal direction of the syringe outer cylinder relative to the first convex portion are provided on a distal portion of an outer peripheral surface of the syringe outer cylinder, a displacement of the distal member from a first state in which the distal member is temporarily mounted on the distal portion of the syringe outer cylinder to a second state in which the accommodation chamber is sealed by the distal member is completed, the first state is a state in which the first convex portion is fitted into the first concave portion and the accommodation chamber is in communication with an outside via a gap between the syringe outer cylinder and the cylindrical portion or a gap provided in the distal member, and the second state is a state in which the first convex portion is fitted into the second concave portion and the second convex portion is fitted into the first concave portion.
(13) A fourth aspect of the present invention is a method for manufacturing a prefilled syringe, and the method includes: an insertion step of inserting a gasket into a syringe outer cylinder; a liquid accommodation step of filling an accommodation chamber communicating with a distal opening of the syringe outer cylinder with a liquid after the insertion step; a temporary mounting step of temporarily mounting a distal member on a distal portion of the syringe outer cylinder such that the accommodation chamber communicates with an outside; a freezing step of freezing the liquid in the accommodation chamber of the syringe outer cylinder on which the distal member is temporarily mounted to obtain a freezing agent; a drying step of drying the freezing agent in a decompression chamber to obtain a freeze-dried agent; and a sealing step of sealing the accommodation chamber by blocking communication between the accommodation chamber and the outside by pushing the distal member into the syringe outer cylinder after the drying step.
(14) The method for manufacturing a prefilled syringe according to item (13), in which the distal member includes a cylindrical portion configured to allow the distal portion of the syringe outer cylinder to be inserted therein, and a nozzle located inside the cylindrical portion and having a lumen communicating with the accommodation chamber, a distal opening of the nozzle is sealed by a cap mounted on the distal member, a first convex portion and a second convex portion located in a proximal direction of the syringe outer cylinder relative to the first convex portion are provided on a distal portion of an outer peripheral surface of the syringe outer cylinder, a first concave portion and a second concave portion located in a distal direction of the syringe outer cylinder relative to the first concave portion are provided in an inner peripheral surface of the cylindrical portion, in the temporary mounting step, in a state in which the first convex portion is fitted into the first concave portion, the accommodation chamber communicates with the outside via a gap between the syringe outer cylinder and the cylindrical portion or a gap provided in the distal member, and in the sealing step, the first convex portion is fitted into the second concave portion, and the second convex portion is fitted into the first concave portion.

According to the present invention, the syringe outer cylinder with the distal member can be put into the decompression chamber in a state (the first state of the distal member) in which the distal member is temporarily mounted on the syringe outer cylinder. In the first state of the distal member, the accommodation chamber communicates with the outside via a gap. Therefore, since the freezing agent in the accommodation chamber of the syringe outer cylinder can be sublimated when the freezing agent is dried (freeze-dried), the freeze-dried agent can be directly manufactured in the accommodation chamber of the syringe outer cylinder. After the completion of the freeze-drying, the distal member temporarily mounted on the syringe outer cylinder can be brought into the second state to seal the accommodation chamber by the distal member. That is, since it is not necessary to attach the distal member to the distal portion of the syringe outer cylinder in the decompression chamber, a configuration of a manufacturing device (the decompression chamber and the like) of the prefilled syringe can be simplified.

### Brief Description of Drawings

[Fig. 1] Fig. 1 is a perspective view of a prefilled syringe according to an embodiment of the invention.
[Fig. 2] Fig. 2 is a longitudinal cross-sectional view of the prefilled syringe in Fig. 1.
[Fig. 3] Fig. 3 is an exploded perspective view of a distal portion of the prefilled syringe in Fig. 1.
[Fig. 4] Fig. 4 is an enlarged view of the distal portion of the prefilled syringe in Fig. 2.
[Fig. 5] Fig. 5 is a longitudinal cross-sectional view taken along a line V-V in Fig. 4.
[Fig. 6] Fig. 6 is a cross-sectional view taken along a line VI-VI in Fig. 4.
[Fig. 7] Fig. 7 is a cross-sectional view of a flange portion of the prefilled syringe in Fig. 2.
[Fig. 8] Fig. 8 is a flowchart showing a method for manufacturing the prefilled syringe in Fig. 1.
[Fig. 9A] Fig. 9A is a cross-sectional view showing a first insertion step.
[Fig. 9B] Fig. 9B is a cross-sectional view showing a liquid accommodation step.
[Fig. 10] Fig. 10 is a cross-sectional view of a temporary mounting step.
[Fig. 11] Fig. 11 is a cross-sectional view showing a drying step.
[Fig. 12] Fig. 12 is a cross-sectional view showing a sealing step.
[Fig. 13A] Fig. 13A is a cross-sectional view of a liquid agent filling step.
[Fig. 13B] Fig. 13B is a cross-sectional view showing a second insertion step.
[Fig. 14A] Fig. 14A is a diagram showing a state in which a cap of the prefilled syringe is removed.
[Fig. 14B] Fig. 14B is a diagram showing a state in which a needle member is mounted on the distal member.
[Fig. 15] Fig. 15 is a cross-sectional view showing a state in which a liquid agent is introduced into an accommodation chamber.
[Fig. 16] Fig. 16 is a cross-sectional view showing a state in which rotation of a plunger is restricted.
[Fig. 17] Fig. 17 is a cross-sectional view showing a state in which a drug solution is delivered to a living body.

### Description of Embodiments

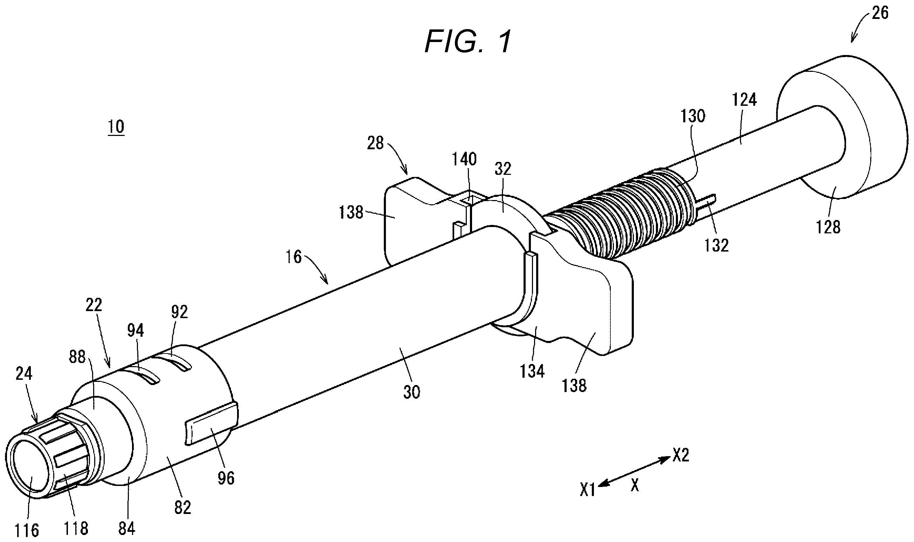

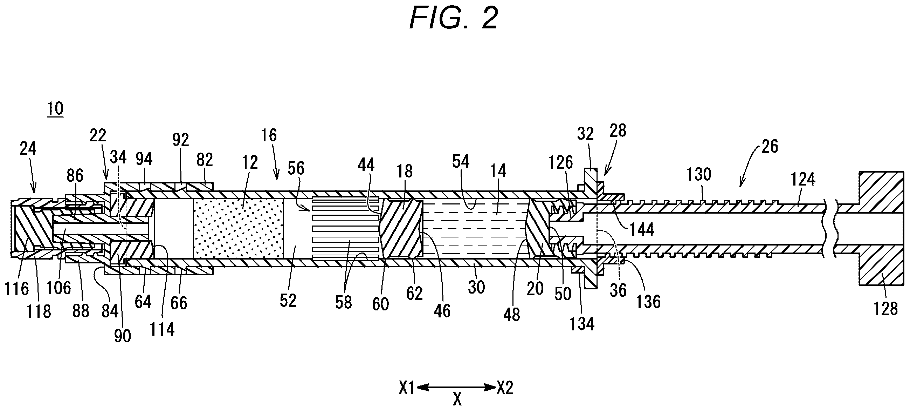

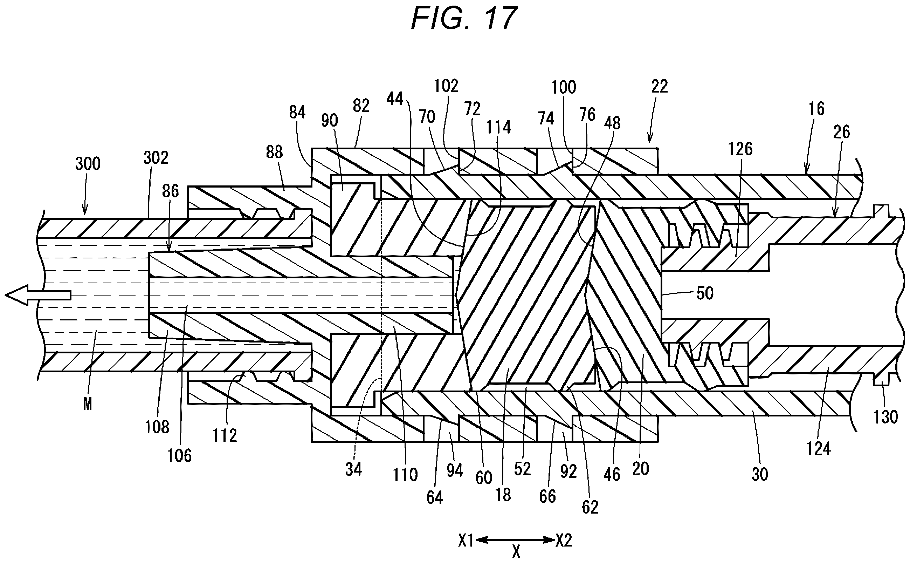

As shown in Figs. 1 and 2, a prefilled syringe 10 according to an embodiment of the invention is a mixing device that mixes a freeze-dried agent 12 and a liquid agent 14 (a dissolving agent) to obtain a drug solution M (see Fig. 17).

The prefilled syringe 10 includes a syringe outer cylinder 16, a first gasket 18, a second gasket 20, a distal member 22, a cap 24, a plunger 26, and a finger grip 28. In the following description, an axial direction of the prefilled syringe 10 may be referred to as an arrow X direction, a distal direction of the prefilled syringe 10 may be referred to as an arrow X1 direction, and a proximal direction of the prefilled syringe 10 may be referred to as an arrow X2 direction.

The syringe outer cylinder 16 is integrally formed of a hard resin material. The syringe outer cylinder 16 has transparency such that an inside thereof can be visually recognized from an outside. The syringe outer cylinder 16 includes a body 30 and a flange portion 32. The body 30 extends along the axial direction (the arrow X direction) of the prefilled syringe 10. The body 30 is formed in a cylindrical shape and has a lumen extending from a distal opening 34 of the syringe outer cylinder 16 to a proximal opening 36 of the syringe outer cylinder 16.

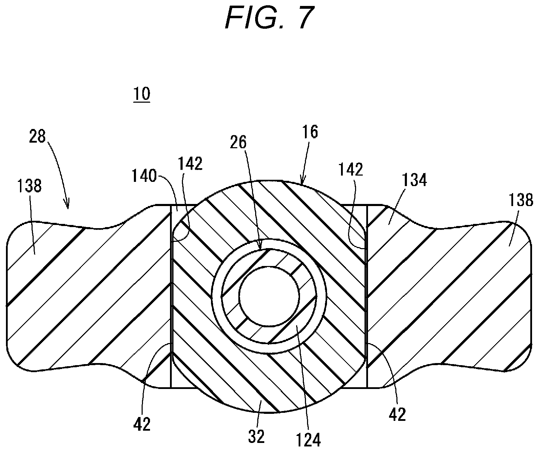

As shown in Fig. 7, the flange portion 32 protrudes outward in a radial direction from a proximal portion of the body 30 and extends in an annular shape. A pair of first restriction portions 42 are provided on a protruding end surface (an outer peripheral surface) of the flange portion 32. The first restriction portion 42 is a flat surface. The pair of first restriction portions 42 face in opposite directions.

In Fig. 2, the first gasket 18 is slidably disposed in the lumen of the syringe outer cylinder 16. The first gasket 18 is formed of, for example, an elastic resin material such as a rubber material or an elastomer material. An outer peripheral surface of the first gasket 18 is in close contact with an inner peripheral surface of the body 30 in a liquid-tight manner. In an initial state of the prefilled syringe 10, the first gasket 18 is located in the middle of the body 30 in the axial direction.

A distal surface (a surface facing the arrow X1 direction) of the first gasket 18 has a first distal slope 44 inclined radially inward in a tapered shape toward the distal direction of the syringe outer cylinder 16. A proximal surface (a surface facing the arrow X2 direction) of the first gasket 18 has a proximal slope 46 inclined radially inward in a tapered shape toward the distal direction of the syringe outer cylinder 16.

The second gasket 20 is slidably disposed in the lumen of the syringe outer cylinder 16. The second gasket 20 is located in the proximal direction (the arrow X2 direction) of the syringe outer cylinder 16 with respect to the first gasket 18. The second gasket 20 is formed of the same material as the first gasket 18. An outer peripheral surface of the second gasket 20 is in close contact with the inner peripheral surface of the body 30 in a liquid-tight manner. In the initial state of the prefilled syringe 10, the second gasket 20 is located at the proximal portion of the body 30.

A distal surface (a surface facing the arrow X1 direction) of the second gasket 20 has a second distal slope 48 inclined radially inward in a tapered shape toward the distal direction of the syringe outer cylinder 16. A taper angle of the second distal slope 48 of the second gasket 20 is the same (substantially the same) as a taper angle of the proximal slope 46 of the first gasket 18. A connection concave portion 50 for connecting the plunger 26 is formed in a proximal surface of the second gasket 20.

In the initial state of the prefilled syringe 10, the syringe outer cylinder 16 has a first chamber 52 located in the distal direction (the arrow X1 direction) with respect to the first gasket 18 and a second chamber 54 located in the proximal direction (the arrow X2 direction) with respect to the first gasket 18. The first chamber 52 is formed between the distal member 22 attached to a distal portion of the syringe outer cylinder 16 and the first gasket 18. The first chamber 52 is an accommodation chamber for accommodating the freeze-dried agent 12.

The second chamber 54 is formed between the first gasket 18 and the second gasket 20. The second chamber 54 is a liquid chamber for accommodating the liquid agent 14 for dissolving the freeze-dried agent 12. In the initial state of the prefilled syringe 10, the first chamber 52 and the second chamber 54 are partitioned by the first gasket 18 without communicating with each other.

The syringe outer cylinder 16 has a bypass structure 56 for communicating the first chamber 52 and the second chamber 54 with each other. The bypass structure 56 includes a plurality of bypass grooves 58 formed in the inner peripheral surface of the body 30. The plurality of bypass grooves 58 is arranged at intervals in a circumferential direction of the body 30. Each bypass groove 58 extends linearly along the axial direction (the arrow X direction) of the body 30. A length of each bypass groove 58 along the arrow X direction is larger than a length of a portion of the first gasket 18 in close contact with the inner peripheral surface of the body 30 along the arrow X direction (a distance from a distal close contact portion 60 to a proximal close contact portion 62).

The number, position, size, shape, and the like of the bypass grooves 58 can be appropriately set. The bypass structure 56 is not limited to a structure including the plurality of bypass grooves 58. For example, a concave bypass flow path may be formed in an inner surface of the body 30 by causing a part of the body 30 to bulge outward in the radial direction.

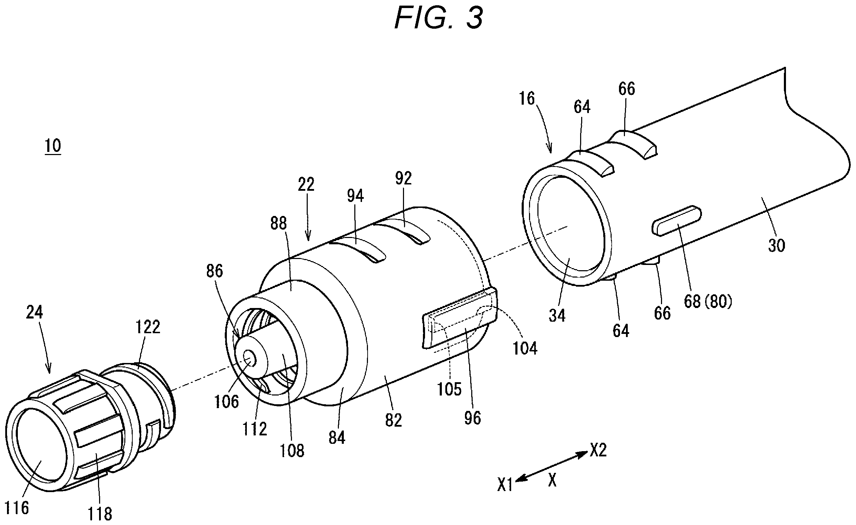

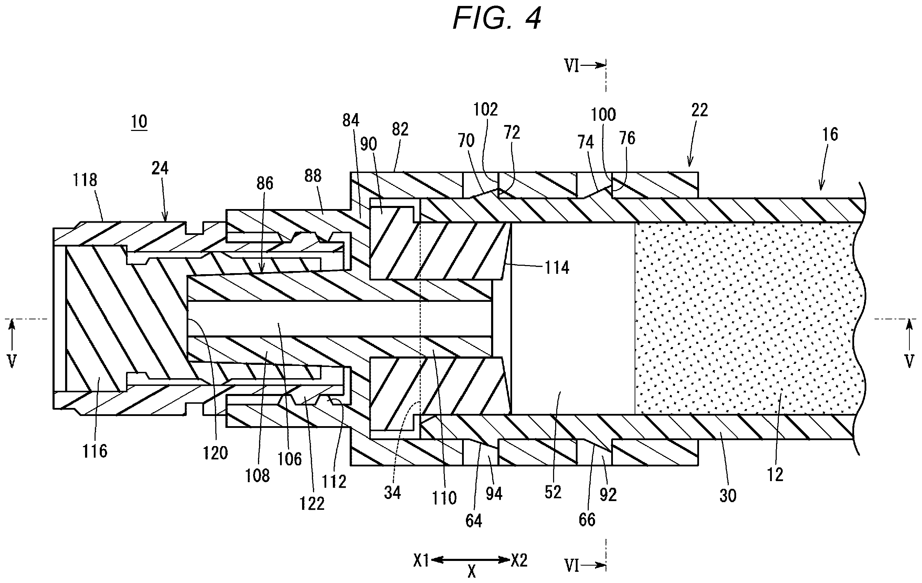

As shown in Figs. 3 to 6, two first convex portions 64, two second convex portions 66, and two first engagement portions 68 protrude from a distal portion of an outer peripheral surface of the body 30. In Figs. 3 and 4, the first convex portion 64 extends along the circumferential direction of the body 30.

As shown in Fig. 4, the first convex portion 64 has a triangular cross section along an axis of the syringe outer cylinder 16. The first convex portion 64 has a first slope 70 and a first stopper surface 72. The first slope 70 is inclined inward from a protruding end of the first convex portion 64 toward the distal direction (the arrow X1 direction) of the syringe outer cylinder 16. The first stopper surface 72 is a flat surface along a direction orthogonal to the axis of the syringe outer cylinder 16, and faces the proximal direction (the arrow X2 direction) of the syringe outer cylinder 16.

In Figs. 3 and 4, the two first convex portions 64 are disposed at an interval in the circumferential direction of the body 30. The two first convex portions 64 are located 180° apart in the circumferential direction of the body 30. The two first convex portions 64 are located at the same position without being displaced from each other in the axial direction of the syringe outer cylinder 16.

A shape and a size of the second convex portion 66 are the same as (substantially the same as) a shape and a size of the first convex portion 64. However, the shape and the size of the second convex portion 66 may be different from the shape and the size of the first convex portion 64.

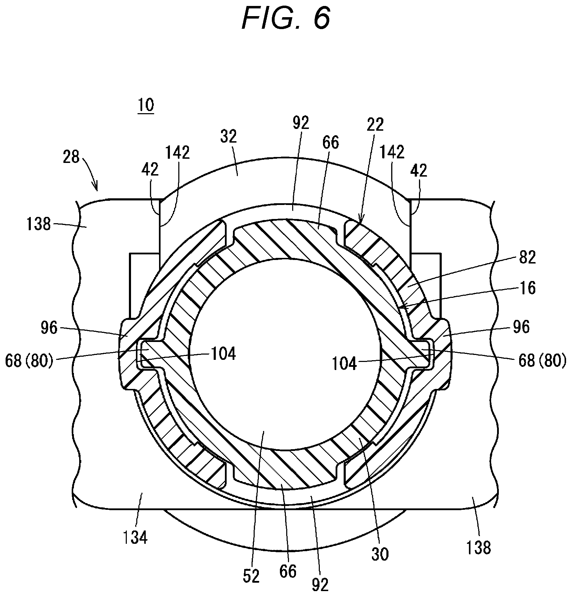

In Figs. 3 and 6, the second convex portion 66 extends along the circumferential direction of the body 30. As shown in Fig. 4, the second convex portion 66 has a triangular cross section along the axis of the syringe outer cylinder 16. The second convex portion 66 has a second slope 74 and a second stopper surface 76. The second slope 74 is inclined inward from a protruding end of the second convex portion 66 toward the distal direction (the arrow X1 direction) of the syringe outer cylinder 16. The second stopper surface 76 is a flat surface along the direction orthogonal to the axis of the syringe outer cylinder 16, and faces the proximal direction (the arrow X2 direction) of the syringe outer cylinder 16.

As shown in Figs. 3, 4, and 6, the two second convex portions 66 are disposed at an interval in the circumferential direction of the body 30. Specifically, the two second convex portions 66 are located 180° apart in the circumferential direction of the body 30. The two second convex portions 66 are located at the same position without being displaced from each other in the axial direction of the syringe outer cylinder 16. The two second convex portions 66 are located at the same positions as the two first convex portions 64 in the circumferential direction of the body 30. The second convex portion 66 is located in the arrow X2 direction with respect to the first convex portion 64.

In Figs. 3, 5, and 6, the first engagement portion 68 includes a protruding portion 80 protruding outward in the radial direction from the outer peripheral surface of the body 30. The protruding portion 80 extends along the axial direction of the syringe outer cylinder 16 (the body 30). A length of the protruding portion 80 along the circumferential direction of the body 30 is less than a length of each of the first convex portion 64 and the second convex portion 66 along the circumferential direction of the body 30. As shown in Fig. 3, a distal end (an end in the arrow X1 direction) of the protruding portion 80 is located in the arrow X1 direction with respect to the second convex portion 66 and in the arrow X2 direction with respect to the first convex portion 64. A proximal end (an end in the arrow X2 direction) of the protruding portion 80 is located in the arrow X2 direction with respect to the second convex portion 66.

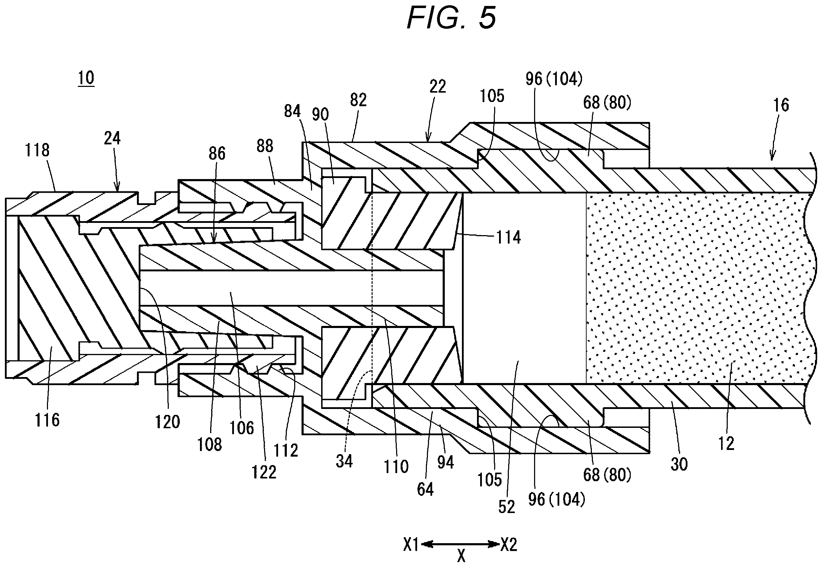

In Figs. 5 and 6, the two first engagement portions 68 are disposed at an interval in the circumferential direction of the body 30. The two first engagement portions 68 are located 180° apart in the circumferential direction of the body 30. The two first engagement portions 68 are located at the same position without being displaced from each other in the axial direction of the syringe outer cylinder 16. The first engagement portion 68 is located between the two second convex portions 66. The first engagement portion 68 is located at a position shifted from the second convex portion 66 by 90° in the circumferential direction of the body 30.

As shown in Figs. 3 and 4, the distal member 22 includes a cylindrical portion 82, an annular wall portion 84, a nozzle 86, a connection section 88, and a packing 90. The cylindrical portion 82, the annular wall portion 84, the nozzle 86, and the connection section 88 are integrally formed of a hard resin material. The cylindrical portion 82 is formed in a cylindrical shape. The distal portion of the syringe outer cylinder 16 (the body 30) is inserted into the cylindrical portion 82. The cylindrical portion 82 is formed with two first concave portions 92, two second concave portions 94, and two second engagement portions 96.

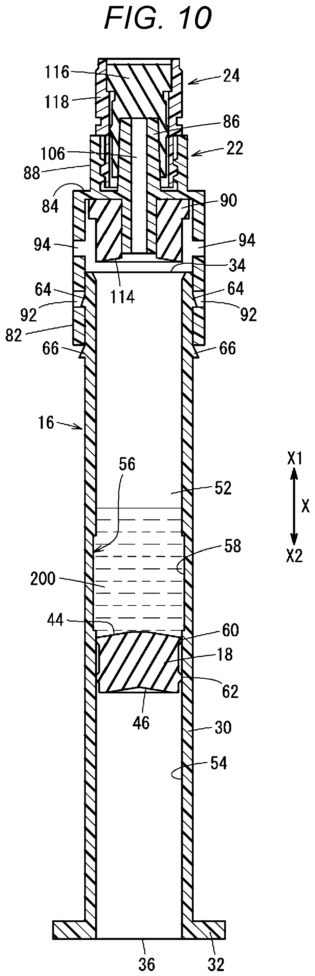

In Figs. 3 and 6, the first concave portion 92 extends along a circumferential direction of the cylindrical portion 82. A length of the first concave portion 92 along the circumferential direction of the cylindrical portion 82 is slightly larger than a length of each of the first convex portion 64 and the second convex portion 66 along the circumferential direction of the body 30. The first concave portion 92 is a rectangular hole penetrating a wall portion of the cylindrical portion 82. However, the first concave portion 92 may be a recess having a bottom portion formed on an inner peripheral surface of the cylindrical portion 82. The first concave portion 92 is formed such that the second convex portion 66 can be fitted thereto. The first concave portion 92 is formed such that the first convex portion 64 can be fitted thereto (see Fig. 10).

An inner surface of the first concave portion 92 has a first abutting surface 100 to which the second stopper surface 76 can abut. The first abutting surface 100 is a flat surface orthogonal to the axial direction (the arrow X direction) of the cylindrical portion 82. The first abutting surface 100 faces the arrow X1 direction.

In Fig. 6, the two first concave portions 92 are disposed at an interval in the circumferential direction of the cylindrical portion 82. The two first concave portions 92 are located 180° apart in the circumferential direction of the cylindrical portion 82. The two first concave portions 92 are located at the same position without being displaced from each other in the axial direction (the arrow X direction) of the cylindrical portion 82.

As shown in Figs. 3 and 4, a shape and a size of the second concave portion 94 are the same (substantially the same) as a shape and a size of the above-described first concave portion 92 penetrating the wall portion of the cylindrical portion 82. However, the shape and the size of the second concave portion 94 may be different from the shape and the size of the first concave portion 92.

The second concave portion 94 extends along the circumferential direction of the cylindrical portion 82. The size, the shape, and the position of the second concave portion 94 are formed such that the first convex portion 64 can be fitted thereto. A length of the second concave portion 94 along the circumferential direction of the cylindrical portion 82 is slightly larger than the length of the first convex portion 64 along the circumferential direction of the body 30. The second concave portion 94 is a rectangular through-hole. However, the second concave portion 94 may be a recess having a bottom portion formed on an inner peripheral surface of the cylindrical portion 82. The second concave portion 94 is formed such that the first convex portion 64 can be fitted thereto.

An inner surface of the second concave portion 94 has a second abutting surface 102 to which the first stopper surface 72 can abut. The second abutting surface 102 is a flat surface orthogonal to the axial direction (the arrow X direction) of the cylindrical portion 82. The second abutting surface 102 faces the arrow X1 direction.

The two second concave portions 94 are disposed at an interval in the circumferential direction of the cylindrical portion 82. The two second concave portions 94 are located 180° apart in the circumferential direction of the cylindrical portion 82. The two second concave portions 94 are located at the same position without being displaced from each other in the axial direction (the arrow X direction) of the cylindrical portion 82**.** The two second concave portions 94 are located at the same positions as the two first concave portions 92 in the circumferential direction of the cylindrical portion 82**.** The second concave portion 94 is located at an interval from the first concave portion 92 in the arrow X1 direction.

In Figs. 3, 5, and 6, the second engagement portion 96 includes a groove portion 104 formed in the inner peripheral surface of the cylindrical portion 82 and a stopper surface 105. The groove portion 104 is formed by the wall portion of the cylindrical portion 82 protruding outward in the radial direction. Therefore, a portion of the cylindrical portion 82 in which the groove portion 104 is formed is not thinned. The groove portion 104 extends along the axial direction of the cylindrical portion 82.

The groove portion 104 extends to a proximal end of the cylindrical portion 82. A shape and a size of the groove portion 104 are formed such that the protruding portion 80 can be inserted. The stopper surface 105 is a surface facing a proximal direction (the arrow X2 direction) of the cylindrical portion 82 in an inner surface of the groove portion 104. The stopper surface 105 is abutted to or close to the distal end of the protruding portion 80.

As shown in Figs. 5 and 6, the two second engagement portions 96 are disposed at an interval in the circumferential direction of the cylindrical portion 82. The two second engagement portions 96 are located 180° apart in the circumferential direction of the cylindrical portion 82. The two second engagement portions 96 are located at the same position without being displaced from each other in the axial direction of the syringe outer cylinder 16.

In Figs. 3 and 4, the annular wall portion 84 protrudes inward in the radial direction from a distal portion of the cylindrical portion 82 and extends in an annular shape. As shown in Fig. 4, the nozzle 86 is connected to a radially inward end portion (inner end portion) of the annular wall portion 84. The nozzle 86 extends along the axial direction (the arrow X direction) of the cylindrical portion 82.

The nozzle 86 is located inside the cylindrical portion 82 to be coaxial with the cylindrical portion 82. The nozzle 86 includes a lumen 106 extending from a distal end to a proximal end of the nozzle 86. The lumen 106 of the nozzle 86 communicates with the first chamber 52 of the body 30. The nozzle 86 includes a distal nozzle portion 108 protruding in the arrow X1 direction from an inner peripheral end portion of the annular wall portion 84 and a proximal nozzle portion 110 protruding in the arrow X2 direction from the inner peripheral end portion of the annular wall portion 84. An outer peripheral surface of the distal nozzle portion 108 is diameter-reduced in a tapered shape toward the arrow X1 direction.

In Figs. 3 and 4, the connection section 88 protrudes from the annular wall portion 84 in the arrow X1 direction and extends in an annular shape. A protruding end of the connection section 88 is located in the arrow X2 direction with respect to a protruding end of the distal nozzle portion 108. A space is formed between an inner peripheral surface of the connection section 88 and an outer peripheral surface of the distal nozzle portion 108. A first screw portion 112 is formed in the inner peripheral surface of the connection section 88. The first screw portion 112 is a female screw portion.

In Fig. 4, the packing 90 is formed of, for example, an elastic resin material such as a rubber material or an elastomer material. The packing 90 is disposed on a surface of the annular wall portion 84 that faces the arrow X2 direction. The packing 90 is in contact with an outer peripheral surface of the proximal nozzle portion 110 in a liquid-tight manner and extends in an annular shape. A proximal surface (a surface facing the arrow X2 direction) of the packing 90 has a slope 114 inclined in a distal direction of the nozzle 86 toward an axis of the nozzle 86. The slope 114 is located in the arrow X2 direction with respect to a protruding end (an end in the arrow X2 direction) of the proximal nozzle portion 110.

As shown in Figs. 3 and 4, the cap 24 includes a sealing member 116 and a cap body 118. The sealing member 116 is formed of a soft rubber material. The sealing member 116 seals a distal opening of the nozzle 86. In Fig. 4, the sealing member 116 is formed with an insertion concave portion 120 opened in the arrow X2 direction. The distal nozzle portion 108 is fitted into the insertion concave portion 120 in liquid-tight and air-tight manners.

The cap body 118 is formed of a hard resin material. The cap body 118 is formed in an annular shape. The sealing member 116 is disposed in a lumen of the cap body 118. The cap body 118 locks the sealing member 116. A second screw portion 122 that is screwed to the first screw portion 112 of the connection section 88 is formed in an outer peripheral surface of a proximal portion (an end portion in the arrow X2 direction) of the cap body 118. The second screw portion 122 is a male screw portion. That is, the cap body 118 is screw-engaged with the distal member 22.

As shown in Figs. 1 and 2, the plunger 26 extends along the axial direction of the syringe outer cylinder 16. The plunger 26 is integrally formed of a hard resin material. The plunger 26 includes a plunger body 124, a distal connection section 126, and a proximal operation portion 128.

In Fig. 2, the plunger body 124 is formed in a cylindrical shape. The plunger body 124 may be formed solid. An outer diameter of the plunger body 124 is less than an inner diameter of the body 30. As shown in Fig. 1, a male screw portion 130 and a rotation restriction portion 132 are provided in an outer peripheral surface of the plunger body 124. The rotation restriction portion 132 is connected to an end portion of the male screw portion 130 in the arrow X2 direction. The rotation restriction portion 132 protrudes outward in the radial direction from the outer peripheral surface of the plunger body 124. The rotation restriction portion 132 extends along an axial direction of the plunger body 124.

In Fig. 2, the distal connection section 126 protrudes from a distal portion of the plunger body 124 in the distal direction (the arrow X1 direction). An outer diameter of the distal connection section 126 is less than the outer diameter of the plunger body 124. The distal connection section 126 is screw-engaged with the second gasket 20 in a state of being inserted into the connection concave portion 50 of the second gasket 20.

The proximal operation portion 128 protrudes outward in the radial direction from a proximal portion of the plunger body 124 and is formed in an annular shape. The proximal operation portion 128 has a size and a shape that allow an easy manual operation. An outer diameter of the proximal operation portion 128 is larger than an outer diameter of the body 30.

As shown in Figs. 1 and 2, the finger grip 28 is provided at a proximal portion of the syringe outer cylinder 16. The finger grip 28 includes a mounting portion 134, a plunger engagement portion 136, and a pair of finger rest portions 138. In Fig. 7, an insertion hole 140 into which the flange portion 32 of the syringe outer cylinder 16 is inserted is formed in the mounting portion 134. A pair of second restriction portions 142 facing the first restriction portions 42 of the flange portion 32 are provided in an inner surface of the insertion hole 140. The pair of second restriction portions 142 are flat surfaces. When the first restriction portions 42 abut to the second restriction portions 142, the finger grip 28 and the syringe outer cylinder 16 are prevented from relatively rotating in the circumferential direction of the plunger body 124.

The numbers, shapes, positions, sizes, and the like of the first restriction portions 42 and the second restriction portions 142 can be appropriately set.

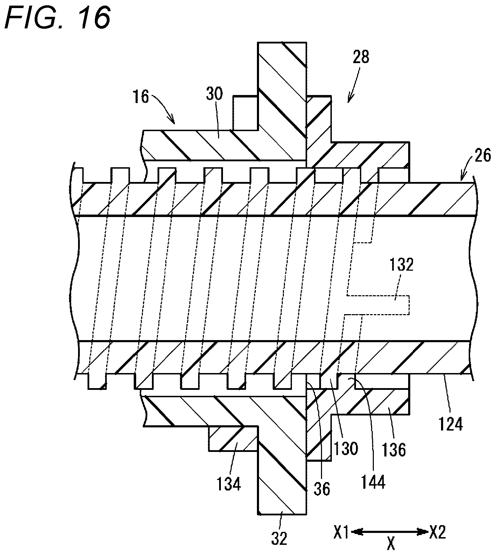

In Figs. 2 and 16, the plunger engagement portion 136 protrudes from the mounting portion 134 in a cylindrical shape in the arrow X2 direction. In an inner peripheral surface of the plunger engagement portion 136, a projection 144 to be screwed with the male screw portion 130 of the plunger 26 is provided. The projection 144 extends by a length less than 360° along the helix of the male screw portion 130 of the plunger 26.

As shown in Fig. 1, the pair of finger rest portions 138 extend in opposite directions from the mounting portion 134. The finger rest portions 138 extend in a flat plate shape.

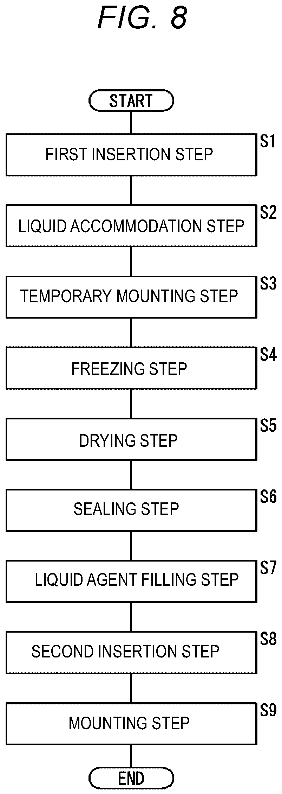

Next, a method for manufacturing the above-described prefilled syringe 10 will be described. As shown in Fig. 8, the method for manufacturing the prefilled syringe 10 includes a first insertion step, a liquid accommodation step, a temporary mounting step, a freezing step, a drying step, a sealing step, a liquid agent filling step, a second insertion step, and a mounting step.

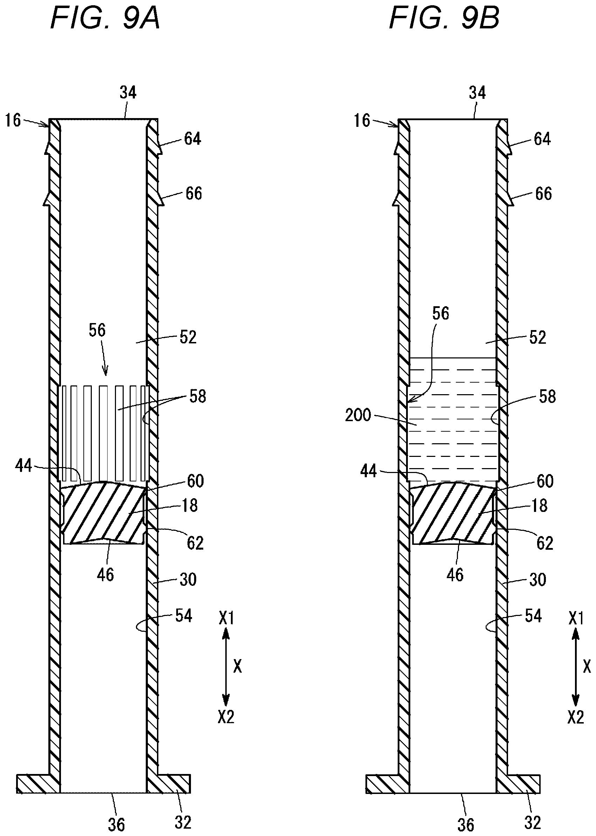

First, in the first insertion step (step S1), as shown in Fig. 9A, the first gasket 18 is inserted into the lumen of the syringe outer cylinder 16. At this time, the first gasket 18 is located in the proximal direction of the syringe outer cylinder 16 relative to the bypass structure 56.

Subsequently, in the liquid accommodation step (step S2 in Fig. 8), as shown in Fig. 9B, a liquid 200 is accommodated in the first chamber 52 of the syringe outer cylinder 16. At this time, the syringe outer cylinder 16 is held in an upright state such that the distal opening 34 of the syringe outer cylinder 16 faces upward.

Next, in the temporary mounting step (step S3 in Fig. 8), as shown in Fig. 10, the distal member 22 with the cap 24 is temporarily mounted on the distal portion of the syringe outer cylinder 16. That is, the distal member 22 is attached from above the syringe outer cylinder 16 such that the distal portion of the syringe outer cylinder 16 is inserted from a proximal opening of the cylindrical portion 82. At this time, the two protruding portions 80 are inserted into the two groove portions 104, thereby positioning the first convex portions 64 and the first concave portions 92 in the circumferential direction of the cylindrical portion 82.

Thereafter, when the distal member 22 with the cap 24 is pressed against the syringe outer cylinder 16, the cylindrical portion 82 moves downward with respect to the syringe outer cylinder 16, and the first convex portions 64 are fitted into the first concave portions 92. Accordingly, the temporary mounting of the distal member 22 to the distal portion of the syringe outer cylinder 16 is completed. In a first state of the distal member 22 in which the distal member 22 is temporarily mounted on the syringe outer cylinder 16, a distal end of the syringe outer cylinder 16 is located in the arrow X2 direction relative to a proximal end of the packing 90. In the first state of the distal member 22, the first chamber 52 of the syringe outer cylinder 16 communicates with an outside via the second concave portions 94 (through-holes) which are gaps.

Note that, when the second concave portion 94 is not a through-hole but a bottomed recess, the first chamber 52 of the syringe outer cylinder 16 may communicate with the outside via a gap (including the groove portions 104) between the syringe outer cylinder 16 and the cylindrical portion 82 in the first state of the distal member 22. When the second concave portion 94 is not a through-hole but a bottomed recess and the distal member 22 is provided with a through-hole different from the second concave portion 94, the first chamber 52 of the syringe outer cylinder 16 may communicate with the outside via the through-hole different from the second concave portion 94 in the first state of the distal member 22. In this case, in the first state of the distal member 22, when the through-hole is located in the distal direction (the arrow X1 direction) relative to the distal end of the syringe outer cylinder 16, the through-hole can be prevented from being blocked by the syringe outer cylinder 16.

Thereafter, in the freezing step (step S4 in Fig. 8), the liquid 200 accommodated in the first chamber 52 is frozen to obtain a freezing agent 204.

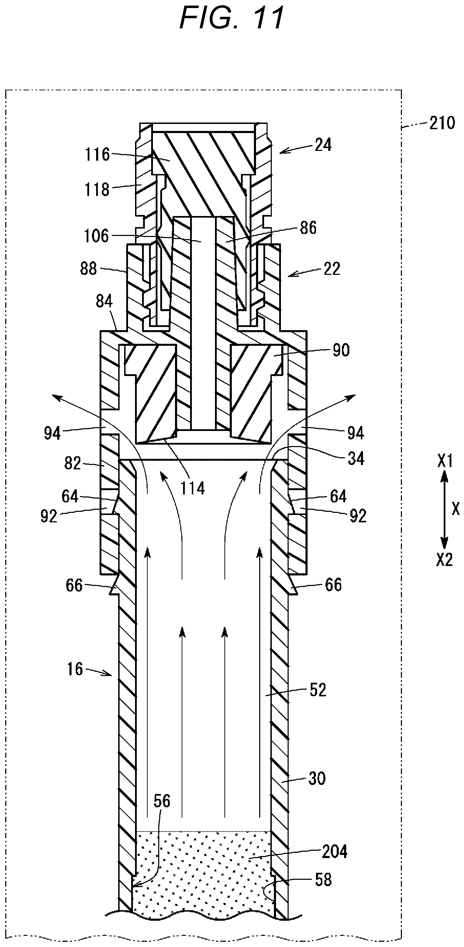

Subsequently, in the drying step (step S5), the freezing agent 204 accommodated in the first chamber 52 is dried (freeze-dried). Specifically, as shown in Fig. 11, the syringe outer cylinder 16 on which the distal member 22 is mounted is disposed in a decompression chamber 210 (a vacuum chamber), and an inside of the decompression chamber 210 is decompressed to a state close to vacuum. Accordingly, since a boiling point of the freezing agent 204 becomes lower than that at the atmospheric pressure, the moisture in the freezing agent 204 is sublimated. At this time, the sublimated moisture (water vapor) is discharged to the outside through the second concave portions 94 (gaps) of the distal member 22. Therefore, the moisture of the freezing agent 204 can be efficiently sublimated. By this drying step, the freeze-dried agent 12 is obtained.

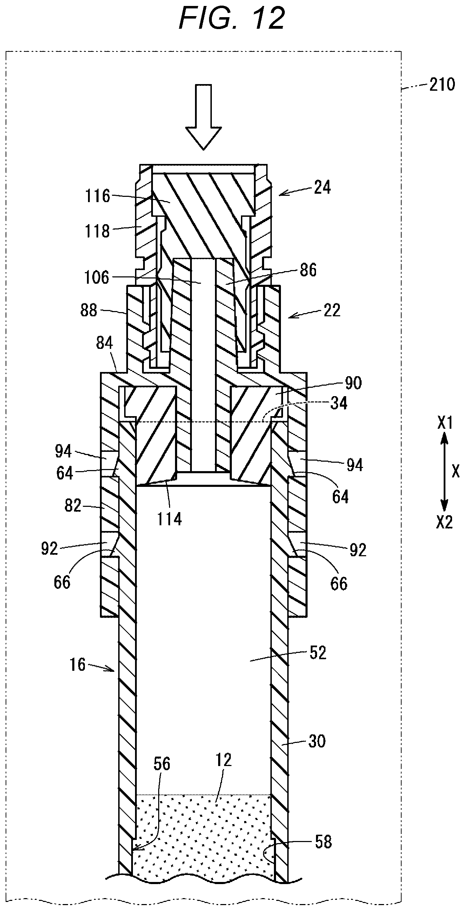

Next, in the sealing step (step S6 in Fig. 8), as shown in Fig. 12, the distal member 22 with the cap 24 is pushed in the arrow X2 direction in the decompression chamber 210. Accordingly, the first convex portions 64 are separated from the first concave portions 92, and the cylindrical portion 82 slides in the arrow X2 direction with respect to the syringe outer cylinder 16. Then, the first convex portions 64 are fitted into the second concave portions 94, and the second convex portions 66 are fitted into the first concave portions 92. That is, the distal member 22 is displaced from the first state to a second state. At this time, since the packing 90 is inserted into the lumen of the syringe outer cylinder 16 in a liquid-tight manner, the first chamber 52 is sealed by the distal member 22 with the cap 24.

In the second state of the distal member 22, an opening portion of the second concave portion 94 that is formed in the inner peripheral surface of the cylindrical portion 82 is blocked by a wall portion of the syringe outer cylinder 16. Further, in the second state of the distal member 22, since the protruding portions 80 are abutted to or close to the stopper surfaces 105 (see Fig. 5), the first convex portions 64 are prevented from being separated from the second concave portions 94, the second convex portions 66 are prevented from being separated from the first concave portions 92, and the cylindrical portion 82 is prevented from sliding in the arrow X2 direction with respect to the syringe outer cylinder 16.

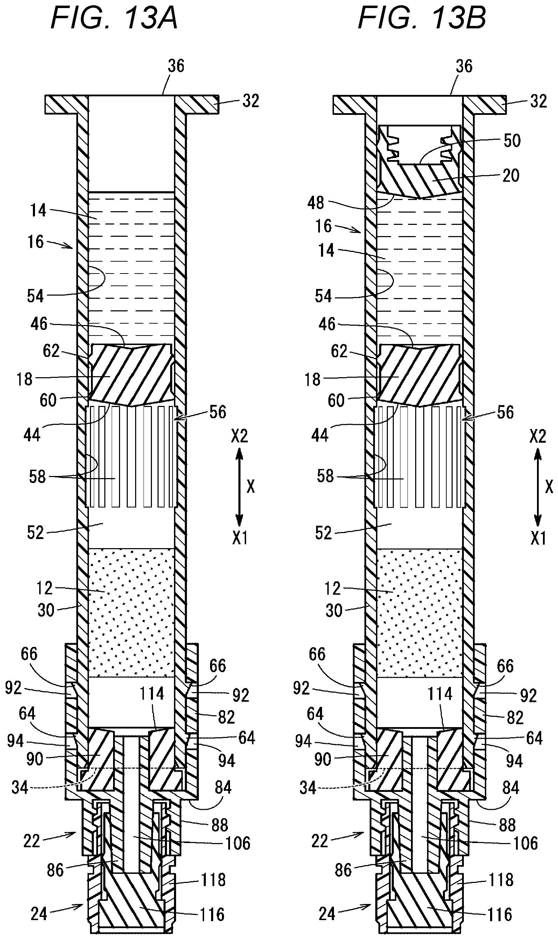

Thereafter, the liquid agent filling step (step S7 in Fig. 8) is performed. Specifically, as shown in Fig. 13A, the syringe outer cylinder 16 is taken out from the decompression chamber 210, and the second chamber 54 is filled with the liquid agent 14 from the proximal opening 36 of the syringe outer cylinder 16 in a state where the syringe outer cylinder 16 is held such that the proximal opening 36 of the syringe outer cylinder 16 faces upward.

Then, in the second insertion step (step S8 in Fig. 8), as shown in Fig. 13B, the second gasket 20 is inserted from the proximal opening 36 of the syringe outer cylinder 16. Note that, the second gasket 20 may be inserted into the syringe outer cylinder 16 by either mechanical plugging or vacuum plugging.

Next, in the mounting step (step S9 in Fig. 8), the finger grip 28 is mounted on the syringe outer cylinder 16, and the plunger 26 is mounted on the second gasket 20 (see Figs. 1 and 2). Accordingly, the prefilled syringe 10 is completed.

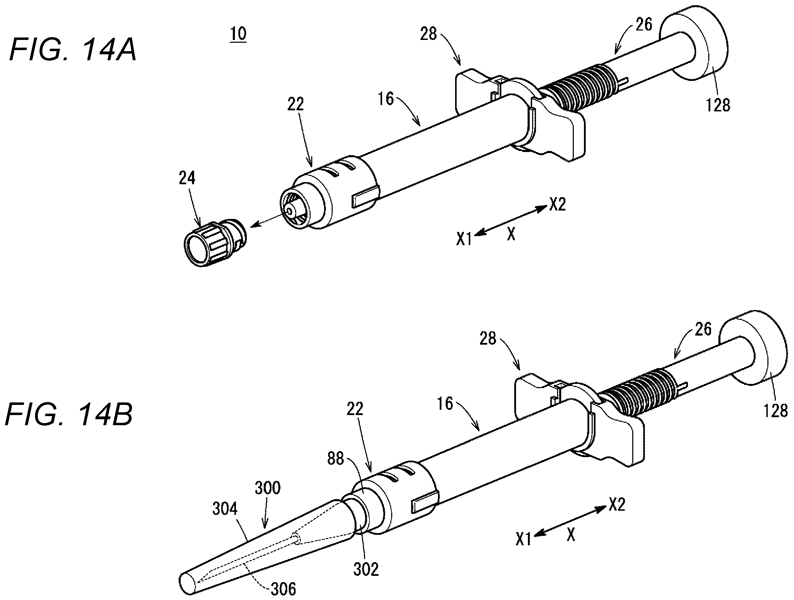

Next, a method for using the prefilled syringe 10 will be described. When using the prefilled syringe 10, as shown in Fig. 14A, a user opens the cap 24 by rotating the cap 24 with respect to the distal member 22. At this time, for example, the user rotates the cap 24 with one hand while holding the syringe outer cylinder 16 or the finger grip 28 with the other hand. Even in such a case, since the protruding portions 80 of the syringe outer cylinder 16 are inserted into the groove portions 104 of the distal member 22 (see Fig. 6), the distal member 22 does not rotate with respect to the syringe outer cylinder 16 together with the cap 24. Since the user does not need to hold the distal member 22 with one hand, an opening operation of the cap 24 can be easily performed. Subsequently, as shown in Fig. 14B, a needle hub 302 of a needle member 300 is mounted on the connection section 88 of the distal member 22.

Thereafter, the user rotates the proximal operation portion 128 with one hand while holding the finger grip 28 or the syringe outer cylinder 16 with the other hand. Accordingly, since the projection 144 of the plunger engagement portion 136 is screwed to the male screw portion 130 of the plunger body 124 (see Fig. 2), the plunger 26 presses the second gasket 20 in the distal direction (the arrow X1 direction) of the syringe outer cylinder 16. Accordingly, the second gasket 20 advances in the arrow X1 direction with respect to the syringe outer cylinder 16.

At this time, since the first restriction portions 42 of the flange portion 32 abut to the second restriction portions 142 of the mounting portion 134 (see Fig. 7), even when the user holds the finger grip 28, the syringe outer cylinder 16 does not rotate together with the plunger 26 due to the frictional force between the second gasket 20 and the syringe outer cylinder 16.

Since the first gasket 18 is pushed in the distal direction (the arrow X1 direction) of the syringe outer cylinder 16 via the liquid agent 14 in the second chamber 54 when the second gasket 20 advances, the first gasket 18 advances in the arrow X1 direction with respect to the syringe outer cylinder 16.

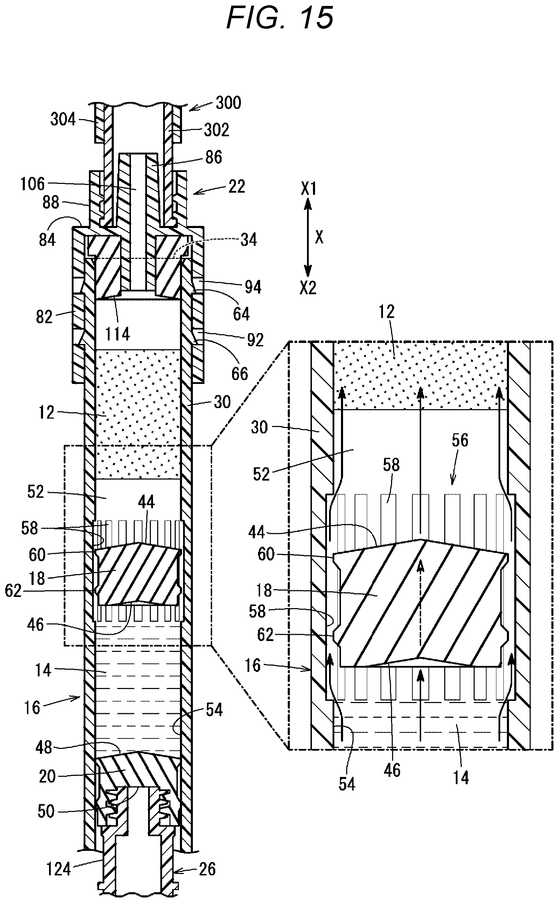

Then, as shown in Fig. 15, the proximal close contact portion 62 of the first gasket 18 is located in the distal direction (the arrow X1 direction) relative to a proximal end of the bypass groove 58, and the distal close contact portion 60 of the first gasket 18 is located in the proximal direction (the arrow X2 direction) relative to a distal end of the bypass groove 58. Accordingly, the liquid agent 14 in the second chamber 54 flows into the first chamber 52 via the plurality of bypass grooves 58.

At this time, the liquid agent 14 in the second chamber 54 is pressed by the second gasket 20 in the distal direction of the syringe outer cylinder 16, and thus smoothly flows into the first chamber 52 via the bypass grooves 58. Thereafter, the second gasket 20 comes into contact with the first gasket 18, and the transition of the liquid agent 14 from the second chamber 54 to the first chamber 52 is completed.

When the transition of the liquid agent 14 from the second chamber 54 to the first chamber 52 is completed, as shown in Fig. 16, since the rotation restriction portion 132 of the plunger 26 abuts to the projection 144 of the plunger engagement portion 136, the rotation of the plunger 26 with respect to the syringe outer cylinder 16 is restricted. Accordingly, the user can easily know that the transfer of the entire liquid agent 14 is completed.

Subsequently, the user mixes the freeze-dried agent 12 and the liquid agent 14 by shaking the distal portion of the syringe outer cylinder 16 from side to side. Accordingly, the freeze-dried agent 12 is dissolved by the liquid agent 14 to prepare the drug solution M.

Thereafter, a protective cap 304 (see Fig. 14B) of the needle member 300 is removed from the needle hub 302, a needle body 306 (see Fig. 14B) is punctured into a patient (a living body), and the plunger 26 is pressed in the distal direction (the arrow X1 direction) of the syringe outer cylinder 16. Accordingly, the drug solution M in the first chamber 52 is administered to the living body through the lumen 106 of the nozzle 86 and a lumen of the needle body 306. Then, when the first distal slope 44 of the first gasket 18 comes into contact with the slope 114 of the packing 90, all the drug solution M in the first chamber 52 is administered to the living body.

The present embodiment has the following effects.

According to the present embodiment, the syringe outer cylinder 16 with the distal member 22 can be put into the decompression chamber 210 in a state (the first state of the distal member 22) in which the distal member 22 is temporarily mounted on the syringe outer cylinder 16. In the first state of the distal member 22, the first chamber 52 (the accommodation chamber) communicates with the outside via a gap. Therefore, since the freezing agent 204 in the first chamber 52 of the syringe outer cylinder 16 can be sublimated when the freezing agent 204 is dried (freeze-dried), the freeze-dried agent 12 can be directly manufactured in the first chamber 52 of the syringe outer cylinder 16. After the completion of the freeze-drying, the distal member 22 temporarily mounted on the syringe outer cylinder 16 can be brought into the second state to seal the first chamber 52 by the distal member 22. That is, since it is not necessary to attach the distal member 22 to the distal portion of the syringe outer cylinder 16 in the decompression chamber 210, a configuration of a manufacturing device (the decompression chamber 210 and the like) of the prefilled syringe 10 can be simplified.

The second concave portion 94 is a through-hole, and the gap provided in the distal member 22 is the through-hole.

According to such a configuration, in the first state of the distal member 22, the freezing agent 204 can be efficiently sublimated via the through-hole (the second concave portion 94) which is a gap.

The first engagement portion 68 is provided at the distal portion of the outer peripheral surface of the syringe outer cylinder 16. The inner peripheral surface of the cylindrical portion 82 is provided with the second engagement portion 96 that engages with the first engagement portion 68 when the distal member 22 is mounted on the syringe outer cylinder 16. One of the first engagement portion 68 and the second engagement portion 96 includes the protruding portion 80. The other of the first engagement portion 68 and the second engagement portion 96 includes the groove portion 104 into which the protruding portion 80 is inserted and which extends along the axial direction of the syringe outer cylinder 16.

According to such a configuration, by inserting the protruding portion 80 into the groove portion 104 when the cylindrical portion 82 is mounted (temporarily mounted) on the distal portion of the syringe outer cylinder 16, the first convex portion 64 and the first concave portion 92 can be positioned in the circumferential direction of the syringe outer cylinder 16. The rotation of the distal member 22 with respect to the syringe outer cylinder 16 can be prevented by the protruding portion 80 and the groove portion 104. Accordingly, for example, even when the cap 24 screw-engaged with the distal member 22 is removed, it is possible to prevent the distal member 22 and the cap 24 from rotating together with respect to the syringe outer cylinder 16. Even when the needle member 300 is mounted on the distal member 22 by screw engagement, it is possible to prevent the distal member 22 and a medical device from rotating together with respect to the syringe outer cylinder 16.

The inner surface of the groove portion 104 has the stopper surface 105 facing the axial direction of the syringe outer cylinder 16. The protruding portion 80 abuts to or is close to the stopper surface 105.

According to such a configuration, even when the distal member 22 is pushed into the syringe outer cylinder 16 in the axial direction of the syringe outer cylinder 16, since the protruding portion 80 abuts to the stopper surface 105, it is possible to prevent the first convex portion 64 from being separated from the second concave portion 94 and the second convex portion 66 from being separated from the first concave portion 92.

The distal member 22 includes the tubular connection section 88 that covers a distal portion of the nozzle 86 from radially outside. The connection section 88 includes the first screw portion 112. The cap 24 includes the second screw portion 122 to be screwed to the first screw portion 112, and the sealing member 116 that seals the distal opening of the nozzle 86 in a state in which the second screw portion 122 is fastened to the first screw portion 112.

According to such a configuration, the distal opening of the nozzle 86 can be effectively sealed by the sealing member 116 with a simple configuration.

A proximal portion of the nozzle 86 is located in the lumen of the cylindrical portion 82. The distal member 22 includes the annular packing 90 disposed in a liquid-tight manner on the outer peripheral surface of the nozzle 86. The packing 90 is inserted into the lumen of the syringe outer cylinder 16 such that the distal opening 34 of the syringe outer cylinder 16 is sealed.

According to such a configuration, the first chamber 52 of the syringe outer cylinder 16 can be effectively sealed by the packing 90.

The proximal surface of the packing 90 has the slope 114 inclined in the distal direction of the nozzle 86 toward the axis of the nozzle 86. The distal surface of the first gasket 18 has the first distal slope 44 inclined radially inward toward the distal direction of the syringe outer cylinder 16.

According to such a configuration, an amount of the drug solution M remaining in the first chamber 52 when the administration of the drug solution M to the living body is completed can be reduced.

The first distal slope 44 of the first gasket 18 can come into contact with the entire slope 114 of the packing 90.

According to such a configuration, the amount of the drug solution M remaining in the first chamber 52 when the administration of the drug solution M to the living body is completed can be further reduced.

The prefilled syringe 10 includes the first gasket 18, the second gasket 20, and the plunger 26. The second gasket 20 is disposed in the lumen of the syringe outer cylinder 16 in the proximal direction relative to the first gasket 18 and is slidable in the axial direction of the syringe outer cylinder 16. The plunger 26 presses the second gasket 20 in the distal direction. In the initial state of the prefilled syringe 10, the second chamber 54 (a liquid chamber) filled with the liquid agent 14 is formed between the first gasket 18 and the second gasket 20. The syringe outer cylinder 16 has the bypass structure 56 for guiding the liquid agent 14 to the first chamber 52. The liquid agent 14 is prevented from flowing into the bypass structure 56 by the first gasket 18 in the initial state of the prefilled syringe 10, and is guided to the first chamber 52 when the first gasket 18 moves in the distal direction of the syringe outer cylinder 16 and the first chamber 52 and the second chamber 54 communicate with each other via the bypass structure 56.

According to such a configuration, the drug solution M can be obtained by guiding the liquid agent 14 from the second chamber 54 to the first chamber 52 via the bypass structure 56 and mixing the liquid agent 14 and the freeze-dried agent 12 in the first chamber 52.

The syringe outer cylinder 16 includes the tubular body 30 and the flange portion 32 that protrudes outward in the radial direction from the proximal portion of the body 30 and extends in the circumferential direction. The prefilled syringe 10 includes the finger grip 28 that is provided at the proximal portion of the syringe outer cylinder 16 and allows the user to hook fingers thereon. The male screw portion 130 is formed on the outer peripheral surface of the plunger 26. The flange portion 32 includes the first restriction portion 42. The finger grip 28 includes the second restriction portions 142 that restrict the rotation of the finger grip 28 in the circumferential direction with respect to the syringe outer cylinder 16 by abutment of the first restriction portions 42 to the second restriction portions 142, and the plunger engagement portion 136 having the projection 144 to be screwed to the male screw portion 130. The plunger 26 moves in the distal direction with respect to the syringe outer cylinder 16 by rotating in the circumferential direction of the syringe outer cylinder 16.

According to such a configuration, the plunger 26 can be easily operated by the finger grip 28. The first restriction portions 42 and the second restriction portions 142 can prevent the finger grip 28 and the syringe outer cylinder 16 from rotating together when the plunger 26 is rotated.

The prefilled syringe according to the present invention is not limited to an example including a so-called dual-chamber type syringe outer cylinder including an accommodation chamber (a first chamber) and a liquid chamber (a second chamber). The prefilled syringe may include a so-called single-chamber type syringe outer cylinder including an accommodation chamber (a first chamber) and not including a liquid chamber (a second chamber). In this case, for example, the liquid agent accommodated in the vial can be aspirated (introduced) into the accommodation chamber of the prefilled syringe through the lumen of the nozzle, thereby mixing the freeze-dried agent and the liquid agent.

Note that, the present invention is not limited to the above-described disclosure, and various configurations can be adopted without departing from the gist of the present invention.

## Claims

1. A prefilled syringe comprising:
a syringe outer cylinder;
a gasket slidably disposed in the syringe outer cylinder;
a freeze-dried agent accommodated in an accommodation chamber of the syringe outer cylinder;
a distal member mounted on a distal portion of the syringe outer cylinder; and
a cap configured to be attached to and detached from the distal member, wherein
the distal member comprises
a cylindrical portion into which the distal portion of the syringe outer cylinder is inserted, and
a nozzle located inside the cylindrical portion and having a lumen communicating with the accommodation chamber,
the cap seals a distal opening of the nozzle in a state of being mounted on the distal member,
a first convex portion and a second convex portion located in a proximal direction of the syringe outer cylinder relative to the first convex portion are provided on a distal portion of an outer peripheral surface of the syringe outer cylinder,
a first concave portion and a second concave portion located in a distal direction of the syringe outer cylinder relative to the first concave portion are provided in an inner peripheral surface of the cylindrical portion,
a displacement of the distal member from a first state in which the distal member is temporarily mounted on the distal portion of the syringe outer cylinder to a second state in which the accommodation chamber is sealed by the distal member is completed,
the first state is a state in which the first convex portion is fitted into the first concave portion and the accommodation chamber is in communication with an outside via a gap between the syringe outer cylinder and the cylindrical portion or a gap provided in the distal member, and
the second state is a state in which the first convex portion is fitted into the second concave portion and the second convex portion is fitted into the first concave portion.

2. The prefilled syringe according to claim 1, wherein
the second concave portion is a through-hole, and
the gap provided in the distal member is the through-hole.

3. The prefilled syringe according to claim 1, wherein
a first engagement portion is provided on the distal portion of the outer peripheral surface of the syringe outer cylinder,
a second engagement portion configured to engage with the first engagement portion when the distal member is mounted on the syringe outer cylinder is provided in the inner peripheral surface of the cylindrical portion,
one of the first engagement portion and the second engagement portion comprises a protruding portion, and
the other of the first engagement portion and the second engagement portion comprises a groove portion that is configured to allow the protruding portion to be inserted therein and that extends along an axial direction of the syringe outer cylinder.

4. The prefilled syringe according to claim 3, wherein
an inner surface of the groove portion has a stopper surface that faces the axial direction of the syringe outer cylinder, and
the protruding portion is abutted to or close to the stopper surface.

5. The prefilled syringe according to claim 1, wherein
the distal member comprises a tubular connection section that covers a distal portion of the nozzle from radially outside,
the connection section comprises a first screw portion, and
the cap comprises
a second screw portion to be screwed to the first screw portion, and
a sealing member configured to seal the distal opening of the nozzle in a state in which the second screw portion is fastened to the first screw portion.

6. The prefilled syringe according to claim 1, wherein
a proximal portion of the nozzle is located in a lumen of the cylindrical portion,
the distal member comprises an annular packing disposed in a liquid-tight manner on an outer peripheral surface of the nozzle, and
the packing is inserted into a lumen of the syringe outer cylinder to seal a distal opening of the syringe outer cylinder.

7. The prefilled syringe according to claim 6, wherein
a proximal surface of the packing has a slope inclined in a distal direction of the nozzle toward an axis of the nozzle, and
a distal surface of the gasket has a distal slope inclined radially inward toward the distal direction of the syringe outer cylinder.

8. The prefilled syringe according to claim 7, wherein
the distal slope of the gasket is configured to come into contact with the entire slope of the packing.

9. The prefilled syringe according to any one of claims 1 to 8, wherein
the gasket is a first gasket,
the prefilled syringe comprises
a second gasket that is disposed in a proximal direction relative to the first gasket in the lumen of the syringe outer cylinder and that is configured to slide in the axial direction of the syringe outer cylinder, and
a plunger configured to press the second gasket in a distal direction,
in an initial state of the prefilled syringe, a liquid chamber filled with a liquid agent is formed between the first gasket and the second gasket,
the syringe outer cylinder has a bypass structure configured to guide the liquid agent to the accommodation chamber, and
the liquid agent is prevented from flowing into the bypass structure by the first gasket in the initial state of the prefilled syringe, and is guided to the accommodation chamber by movement of the first gasket in the distal direction of the syringe outer cylinder and the accommodation chamber and the liquid chamber communicating with each other via the bypass structure.

10. The prefilled syringe according to claim 9, wherein
the syringe outer cylinder comprises
a tubular body, and
a flange portion protruding radially outward from a proximal portion of the tubular body and extending in a circumferential direction,
the prefilled syringe comprises
a finger grip provided on a proximal portion of the syringe outer cylinder and configured to allow a user to hook a finger thereon,
a male screw portion is formed on an outer peripheral surface of the plunger,
the flange portion comprises a first restriction portion,
the finger grip comprises
a second restriction portion configured to restrict rotation of the syringe outer cylinder in a circumferential direction with respect to the syringe outer cylinder by abutment of the first restriction portion to the second restriction portion, and
a plunger engagement portion having a projection to be screwed to the male screw portion, and
the plunger moves in the distal direction with respect to the syringe outer cylinder by rotating in a circumferential direction of the finger grip.

11. A distal member of a prefilled syringe, the prefilled syringe comprising a syringe outer cylinder, a gasket slidably disposed in the syringe outer cylinder, a freeze-dried agent accommodated in an accommodation chamber of the syringe outer cylinder, and a cap configured to be attached to and detached from the distal member,
a first convex portion and a second convex portion located in a proximal direction of the syringe outer cylinder relative to the first convex portion being provided on a distal portion of an outer peripheral surface of the syringe outer cylinder,
the distal member comprising:
a cylindrical portion into which the distal portion of the syringe outer cylinder is inserted; and
a nozzle located inside the cylindrical portion and having a lumen communicating with the accommodation chamber, wherein
the cap seals a distal opening of the nozzle in a state of being mounted on the distal member,
a first concave portion and a second concave portion located in a distal direction of the syringe outer cylinder relative to the first concave portion are provided in an inner peripheral surface of the cylindrical portion,
a displacement of the distal member from a first state in which the distal member is temporarily mounted on the distal portion of the syringe outer cylinder to a second state in which the accommodation chamber is sealed by the distal member is completed,
the first state is a state in which the first convex portion is fitted into the first concave portion and the accommodation chamber is in communication with an outside via a gap between the syringe outer cylinder and the cylindrical portion or a gap provided in the distal member, and
the second state is a state in which the first convex portion is fitted into the second concave portion and the second convex portion is fitted into the first concave portion.

12. A syringe outer cylinder of a prefilled syringe, wherein
the prefilled syringe comprises a distal member mounted on a distal portion of the syringe outer cylinder, a gasket slidably disposed in the syringe outer cylinder, a freeze-dried agent accommodated in an accommodation chamber of the syringe outer cylinder, and a cap configured to be attached to and detached from the distal member,
the distal member comprises
a cylindrical portion into which the distal portion of the syringe outer cylinder is inserted, and
a nozzle located inside the cylindrical portion and having a lumen communicating with the accommodation chamber,
the cap seals a distal opening of the nozzle in a state of being mounted on the distal member,
a first concave portion and a second concave portion located in a distal direction of the syringe outer cylinder relative to the first concave portion are provided in an inner peripheral surface of the cylindrical portion,
a first convex portion and a second convex portion located in a proximal direction of the syringe outer cylinder relative to the first convex portion are provided on a distal portion of an outer peripheral surface of the syringe outer cylinder,
a displacement of the distal member from a first state in which the distal member is temporarily mounted on the distal portion of the syringe outer cylinder to a second state in which the accommodation chamber is sealed by the distal member is completed,
the first state is a state in which the first convex portion is fitted into the first concave portion and the accommodation chamber is in communication with an outside via a gap between the syringe outer cylinder and the cylindrical portion or a gap provided in the distal member, and
the second state is a state in which the first convex portion is fitted into the second concave portion and the second convex portion is fitted into the first concave portion.

13. A method for manufacturing a prefilled syringe, the method comprising:
an insertion step of inserting a gasket into a syringe outer cylinder;
a liquid accommodation step of filling an accommodation chamber communicating with a distal opening of the syringe outer cylinder with a liquid after the insertion step;
a temporary mounting step of temporarily mounting a distal member on a distal portion of the syringe outer cylinder such that the accommodation chamber communicates with an outside;
a freezing step of freezing the liquid in the accommodation chamber of the syringe outer cylinder on which the distal member is temporarily mounted to obtain a freezing agent;
a drying step of drying the freezing agent in a decompression chamber to obtain a freeze-dried agent; and
a sealing step of sealing the accommodation chamber by blocking communication between the accommodation chamber and the outside by pushing the distal member into the syringe outer cylinder after the drying step.

14. The method for manufacturing a prefilled syringe according to claim 13, wherein
the distal member comprises
a cylindrical portion configured to allow the distal portion of the syringe outer cylinder to be inserted therein, and
a nozzle located inside the cylindrical portion and having a lumen communicating with the accommodation chamber,
a distal opening of the nozzle is sealed by a cap mounted on the distal member,
a first convex portion and a second convex portion located in a proximal direction of the syringe outer cylinder relative to the first convex portion are provided on a distal portion of an outer peripheral surface of the syringe outer cylinder,
a first concave portion and a second concave portion located in a distal direction of the syringe outer cylinder relative to the first concave portion are provided in an inner peripheral surface of the cylindrical portion,
in the temporary mounting step, in a state in which the first convex portion is fitted into the first concave portion, the accommodation chamber communicates with the outside via a gap between the syringe outer cylinder and the cylindrical portion or a gap provided in the distal member, and
in the sealing step, the first convex portion is fitted into the second concave portion, and the second convex portion is fitted into the first concave portion.
